# EUROPÄISCHE PATENTSCHRIFT

(11) **EP 0 825 191 B1**
(45) Veröffentlichungstag und Bekanntmachung des Hinweises auf die Patenterteilung: **27.02.2002**
(21) Anmeldenummer: 97113944.9
(22) Anmeldetag: 13.08.1997
(51) Int. Cl.: C07D 407/12, A61K 31/35, C07F 9/6558

(54) **Chromanyl-ascorbinsäurederivate, deren Herstellung und Verwendung**
Chromanyl-ascorbic acid derivatives, their preparation and use
Dérivés chromanylés d'acide ascorbique, leur préparation et utilisation

(30) Priorität: 21.08.1996 DE 19633560
(43) Veröffentlichungstag der Anmeldung: 25.02.1998
(73) Patentinhaber: BASF AKTIENGESELLSCHAFT, 67056 Ludwigshafen (DE)
(72) Erfinder: Rosenau, Thomas, Dr., 99817 Eisenach (DE); Habicher, Wolf-Dieter, Dr., 01217 Dresden (DE); Streicher, Harald, Dr., 67069 Ludwigshafen (DE)

(56) Entgegenhaltungen:
- EP-A- 0 436 936
- JP-A- 4 099 772
- DATABASE WPI Week 199219, Derwent Publications Ltd., London, GB; Class B02, AN 1992-157346 & JP 04 099 772 A (KANEBO LTD) 31 M{rz 1992

## Beschreibung

Die Erfindung betrifft Chromanyl-ascorbinsäurederivate der allgemeinen Formel I 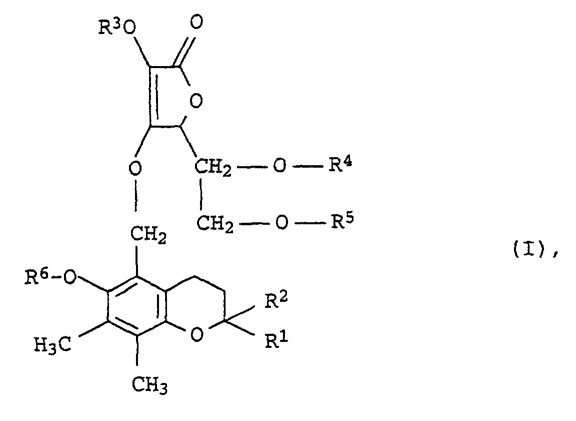 in der
- R¹: für einen Kohlenwasserstoffrest mit 1 bis 4 C-Atomen steht,
- R²: für einen linearen oder Methyl-verzweigten gesättigten oder ungesättigten aliphatischen Kohlenwasserstoffrest mit 12 bis 24 C-Atomen steht,
- R³: für Wasserstoff, eine Alkyl- oder Acylgruppe mit 1 bis 4 C-Atomen, den -PO₃H₂-Rest oder einen Glycosidylrest steht,
- R⁴ und R⁵: jeweils für Wasserstoff, eine Alkyl- oder Acylgruppe mit 1 bis 4 C-Atomen stehen,
- R⁶: für Wasserstoff oder eine Acylgruppe der Formel -CO-R⁷ steht und
- R⁷: für einen gesättigten oder ungesättigten aliphatischen Rest mit 1 bis 20 C-Atomen oder den Phenylvinylrest steht.

Bevorzugte Chromanyl-ascorbinsäurederivate der allgemeinen Formel I sind solche, in denen
- R¹: für eine Methylgruppe steht,
- R²: für den Phytylrest 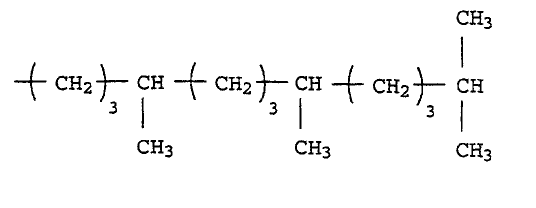 steht und R³, R^{4,} R⁵ und R⁶ jeweils für Wasserstoff stehen, d.h. ein in 5a-Position von Tocopherol mit Ascorbinsäure verknüpftes Produkt der Formel Ia

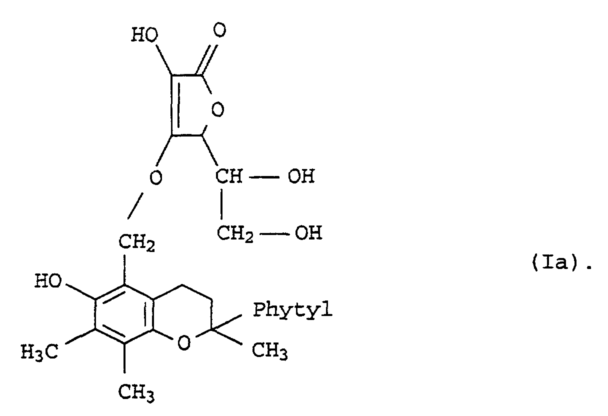

Sowohl das Chromanderivat α-Tocopherol (Vitamin E) als auch Ascorbinsäure (Vitamin C) und Ascorbinsäurederivate spielen aufgrund ihrer oxidationsinhibierenden und radikalfangenden Wirkung in biologischen Systemen eine wichtige Rolle und werden in vielfältiger Weise für pharmazeutische oder kosmetische Zwecke eingesetzt. Dabei wirkt Vitamin E vorrangig als Radikalfänger in lipophilen Phasen und Vitamin C und andere Ascorbinsäurederivate aufgrund ihrer polaren Struktur in wäßrigen Phasen als Reduktionsmittel. Beide Verbindungsklassen werden häufig gemeinsam eingesetzt, da sie sich in ihrer Wirkung synergistisch ergänzen. Die stark unterschiedliche Polarität der Chromanderivate, wie Vitamin E, einerseits und der Ascorbinsäurederivate, wie Vitamin C, andererseits verhindern jedoch eine optimale Wechselwirkung und die volle Entfaltung des Wirkungspotentials bei der Verwendung von Gemischen beider Verbindungen. Auch physikalische Erscheinungen wie Diffusionsvorgänge, Teilchengröße und Teilchenverteilung können die Wirksamkeit stark beeinträchtigen. So wird bei kommerziellen Vitamin-E-Präparaten z.Z. nur ein kleiner Bruchteil der verabreichten Dosis ausgenutzt.

Die Verwendung von Vitamin E zur Stabilisierung von Kunststoffen gegenüber den schädigenden Einflüssen von Sauerstoff, Wärme und Licht ist z.B. aus DE-A 11 36 100 und DE-A 11 14 319 und der EP-A 36 169 bekannt. Auch die Stabilisierung von Fetten und Ölen in Nahrungsmitteln durch Vitamin E ist beschrieben (vgl. Ullmanns Encyklopädie der technischen Chemie, Band 23, Seite 649).

Die bekannten Chromanderivate lassen jedoch bezüglich der Wirksamkeit, der Löslichkeit in hydrophilen Systemen, der Verteilung bzw. der Dispergierbarkeit in vivo sowie in der Flüchtigkeit noch zu wünschen übrig.

Der vorliegenden Erfindung lag daher die Aufgabe zugrunde, neue Chromanderivate zur Verfügung zu stellen, die die obengenannten Nachteile der bekannten Verbindungen nicht oder nur in geringerem Ausmaß aufweisen.

Insbesondere war es die Aufgabe der Erfindung, ein neues körperverträgliches Vitamin-E-Derivat zur Verfügung zu stellen, mit dessen Hilfe es gelingt, die Resorbierbarkeit von Vitamin E im menschlichen und tierischen Organismus zu verbessern.

Vitamin E wird im menschlichen Organismus im Darmtrakt resorbiert. Bei kommerziellen Vitamin-E-Präparaten werden derzeit nur etwa 10 % der verabreichten Vitamin-E-dosis resorbiert, der Rest unverändert ausgeschieden. Ein wesentlicher Grund hierfür ist die starke Koagulation der Präparate im basischen Darmmedium.

Im menschlichen Organismus besteht zwischen Magen und Darmtrakt ein deutliches pH-Gefälle. Während der pH-Wert im Magen unterhalb von 3 liegt, also im sauren Bereich, herrscht im Darmtrakt ein basisches Medium (pH > 9). Um die Resorbierbarkeit von Vitamin E zu erhöhen, muß der Wirkstoff wenigstens kurzzeitig in feiner Verteilung vorliegen. Da dies auf physikalischem Wege schwer zu verwirklichen ist, war es eine besondere Aufgabe der Erfindung, ein Vitamin-E-Derivat zur Verfügung zu stellen, aus dem Vitamin E im Darmtrakt in feiner Verteilung chemisch freigesetzt wird, wobei alle bei diesem Vorgang beteiligten oder gebildeten Stoffe natürlich biologisch unbedenklich sein müssen.

Überraschenderweise ist es gelungen, Vitamin E in 5a-Position mit Vitamin C zu verknüpfen. Die resultierende Verbindung der Formel Ia ist in saurem Milieu (z.B. im Magen) stabil. In basischem Medium (z.B. im Darmtrakt) dagegen wird ein Ascorbatanion unter Ausbildung eines ortho-Chinonmethids aus der Verbindung der Formel Ia eliminiert. Das Ascorbatanion reduziert dann das intermediäre ortho-Chinonmethid zu Vitamin E, das jetzt in feiner Verteilung vorliegt und daher gut resorbierbar ist. Die Ausbeute an "ausgefälltem" Vitamin E beträgt etwa 80 %, bezogen auf eingesetzte Verbindung der Formel Ia und kann durch Zugabe von weiterem Vitamin C, also von Vitamin C in nicht an Vitamin E gebundener Form, weiter erhöht werden.

Bei dieser durch Basen induzierten Spaltung der Verbindung der Formel Ia werden neben den als Hauptprodukten gebildeten Verbindungen: feinverteiltes Vitamin E und Vitamin C in oxidierter Form, nur noch para-Tocopherylchinon und nicht oxidiertes Vitamin C freigesetzt. Da para-Tocopherylchinon auch normalerweise als Abbauprodukt von Vitamin E im Körper vorkommt, sind alle aus dem Verknüpfungsprodukt der Formel Ia freigesetzten Stoffe biologisch kompatibel.

Die Herstellung der Verbindung der Formel Ia sowie der übrigen Chromanyl-ascorbinsäurederivate der allgemeinen Formel I ist relativ unkompliziert.

Gegenstand der Erfindung ist auch ein Verfahren zur Herstellung von Chromanyl-ascorbinsäurederivaten der allgemeinen Formel I, das dadurch gekennzeichnet ist, daß man ein 5-Halogenmethylchroman der allgemeinen Formel II 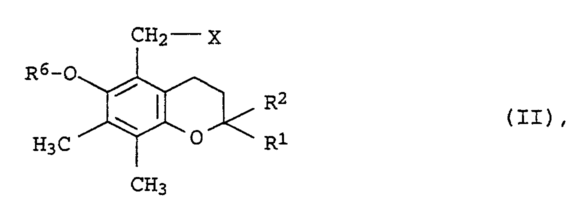 in der R¹, R² und R⁶ die oben angegebene Bedeutung haben und X für Br oder Cl steht, mit einem Alkalisalz oder Erdalkalisalz der Ascorbinsäure selbst oder eines Ascorbinsäurederivates der allgemeinen Formel III 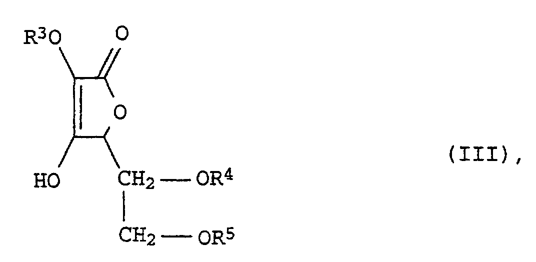 in der R³ bis R⁵ die oben angegebene Bedeutung haben, umsetzt.

Mit besonderem Vorteil gelingt das erfindungsgemäße Verfahren zur Herstellung der Chromanyl-ascorbinsäurederivate der allgemeinen Formel I, insbesondere der Verbindung der Formel Ia, in dem man die Alkalisalze oder Erdalkalisalze der Ascorbinsäurederivate bzw. der Ascorbinsäure selbst in situ, d.h. im Reaktionsgemisch durch die Gegenwart von Basen herstellt und anschließend umsetzt.

Die als Ausgangsverbindungen für das erfindungsgemäße Verfahren verwendeten 5-Halogenmethyl-chromane der allgemeinen Formel II sind bekannte Verbindungen und können beispielsweise selektiv durch Bromieren bzw. Chlorieren der 5-Methyl-Gruppe von Vitamin E bzw. der Chromanderivate in inerten organischen Lösungsmitteln, wie Hexan, Heptan, Octan oder Toluol hergestellt werden. Als 5-Halogenmethyl-chromane der allgemeinen Formel II, in der R⁶ für eine Acylgruppe stehen, kommen insbesondere die entsprechenden Acetate, Palmitate, Sorbate, Oleate, Linoleate und Cinnamate in Betracht.

Zur Durchführung des erfindungsgemäßen Verfahrens geht man im allgemeinen so vor, daß man Ascorbinsäure bzw. ein Ascorbinsäurederivat und ein Alkali- oder Erdalkaliascorbat bzw. Alkali- oder Erdalkalisalz eines Ascorbinsäurederivates oder Alkali- oder Erdalkalihydroxid in einem universellen Lösungsmittel, wie Dimethylsulfoxid unter intensiver Durchmischung in der Wärme löst, die Lösung auf Raumtemperatur abkühlt und dann mit der Lösung von 5a-Brom-α-tocopherol oder einem seiner Salze versetzt und das Reaktionsgemisch unter Inertgasschutz und Rühren für 2 bis 6 Stunden, vorzugsweise 2,5 bis 3,5 Stunden auf Temperaturen von 40 bis 60°C erhitzt. Die Aufarbeitung des Reaktionsgemisches erfolgt auf übliche Weise, beispielsweise durch Extraktion mit n-Hexan und Wasser und anschließende Chromatographie an Al₂O₃. Natriumascorbat und 5a-Halogen-α-tocopherol bzw. dessen Salz verwendet man in etwa äquimolarem Verhältnis. Es hat sich aber als vorteilhaft erwiesen, dem Reaktionsansatz zusätzlich noch Ascorbinsäure zuzusetzen, um Nebenreaktionen, wie die Bildung von Tocopherylchinonen, zu verhindern. Die zusätzliche Menge an Ascorbinsäure beträgt im allgemeinen 0,5 bis 2 Mol pro Mol 5a-Halogen-α-tocopherol.

Mit besonderem Vorteil gestaltet sich das Verfahren, wenn man das 5-Halogenmethyl-chroman der allgemeinen Formel II gleich in der Lösung einsetzt, in der es hergestellt wurde.

Gegenstand der Erfindung ist weiterhin die Verwendung der Chromanyl-ascorbinsäurederivate der allgemeinen Formel I, insbesondere die der Verbindung der Formel Ia, als pharmazeutische oder kosmetische Wirkstoffe oder Nahrungsmittelergänzungsstoffe, bzw. als Bioantioxidantien.

Gegenstand der Erfindung ist weiterhin die Verwendung der Chromanyl-ascorbinsäurederivate der allgemeinen Formel I, insbesondere der Verbindung der Formel Ia, zur Stabilisierung von organischen Stoffen, insbesondere von Nahrungsmitteln, pharmazeutischen oder kosmetischen Präparaten oder Kunststoffen gegen die schädigende Einwirkung von Sauerstoff, Wärme und/oder Licht.

Besonders wirkungsvoll ist die Verwendung der Chromanyl-ascorbinsäurederivate der allgemeinen Formel I, insbesondere der Verbindung der Formel Ia, wenn man sie im Gemisch mit Ascorbinsäure oder Ascorbinsäurederivaten einsetzt.

Das nachstehende Beispiel soll das erfindungsgemäße Verfahren näher erläutern.

### Beispiel 1

In einer 250 ml Flasche, ausgerüstet mit einem effizienten Magnetrührer, wurde eine Mischung aus 2,18 g (11 mmol) trockenem, fein gepulvertem Natriumascorbat, 2,64 g (15 mmol) gepulverter Ascorbinsäure und 50 ml Dimethylsulfoxid (DMS) für 2 Stunden (h) bei 60°C gerührt und anschließend das Reaktionsgemisch auf Raumtemperatur (RT) abgekühlt. Hierzu wurde innerhalb von 2 h unter Inertgasschutz und Rühren eine Lösung von 5,1 g (10 mmol) 5a-Brom-α-tocopherol in einem Gemisch aus 10 ml Tetrahydrofuran (THF) und 10 ml DMS addiert. Danach wurde die Mischung 3 h bei 50°C gerührt und anschließend auf RT abgekühlt. Anschließend wurde das Reaktionsgemisch mit 100 ml n-Hexan und 50 ml Wasser versetzt, extrahiert und die gebildeten Phasen getrennt. Die die Hauptmenge an DMS enthaltende wäßrige Phase wurde mit 10 ml n-Hexan extrahiert und die vereinten organischen Phasen mit je 20 ml Wasser DMS-frei gewaschen. Die organische Phase wurde sorgfältig mit Na₂SO₄ getrocknet und anschließend mit 20 g neutralem Al₂O₃ und 50 ml trockenem n-Hexan versetzt. Die Mischung wurde gerührt bis das Lösungsmittel farblos war. Anschließend wurde das gelbe Al₂O₃ mittels einer Glasfilterfritte abgetrennt und mit 50 ml Diethylether gewaschen bis das Filtrat farblos war. Anschließend wurde das gewünschte Produkt der Formel Ia mit 100 ml Methanol aus dem gelben Al₂O₃ extrahiert. Man erhielt eine methanolische Lösung von reiner 5a-Tocopheryl-ascorbinsäure (Ia), aus der durch Entfernen des Methanols unter vermindertem Druck bei Raumtemperatur 5a-Tocopheryl-ascorbinsäure in reiner Form und einer Ausbeute von 63 % der Theorie, bezogen auf eingesetztes 5a-Brom-α-tocopherol erhalten wurde. Die Struktur, wie sie von Formel Ia dargestellt wird, wurde durch Elementaranalyse sowie ¹³C-NMR, 1H NMR, FAB-MS und MALDI-TOF-MS bestätigt.
¹H-NMR (DMSO-d₆/CDCl₃): δ 2.11 (s. 3H, CH₃-Ar), 2.13 (s. 3H, CH₃-Ar), 2.63 (t, 2H, Ar-CH₂-CH₂), 3.6 (d, 2H, -CH-CH(OH)-CH₂OH), 3.7 (m, 1H, -CH-CH(OH)-CH₂OH), 4.63 (s, 2H, Ar-CH₂-O), 4.77 (d, 1H, -CH-CH(OH)-CH₂OH). Die starke Resonanz der isoprenoiden Seitenkette unterhalb von 2,0 ppm ist nicht aufgezeigt.
¹³C-NMR (DMSO-d₆/CDCl₃) : δ 11.8 (C-8b), 12.1 (C-7a), 19.5 (C-4a'), 19.6 (C-8a'), 20.8 (C-4), 21.0 (C-2'), 22.5 (C-13'), 22.6 (C-12a'), 23.6 (C-2a), 24.5 (C-6'), 24.8 (C-10'), 27.9 (C-12'), 31.6 (C-3), 32.6 (C-8'), 32.7 (C-4'), 37.3 (C-7'), 37.4 (C-5'), 37.5 (C-9'), 37.6 (C-3'), 39.4 (C-11'), 39.8 (C-1'), 61.9 (-CH₂OH), 71.0 (-CH(OH)-CH₂OH), 74.3 (C-2), 77.9 (-CH-CH(OH)-CH₂OH), 115.1 (C^{Ar}), 116.1 C^{Ar}), 119.5 (=C-O-CH₂), 123.0 (C^{Ar}), 125.5 (C^{Ar}), 144.6 (C^{Ar}), 147.3 (C^{Ar}), 156.8 (=C-OH), 173.8 (CO)
MALDI-TOF-MS m/z: 606 (M+H⁻)

Beispiele für die Anwendung von 5a-Tocopheryl-ascorbinsäure als Wirkstoff in kosmetischen Formulierungen (für die kosmetischen Zusatzstoffe wurden INCI-Namen verwendet).

### Beispiel 2

Zusammensetzung für fettfreies Sonnenschutz-Gel

| Massengehalt (Gew.-%) | |
|---|---|
| 0,40 | Acrylate/C₁₀-C₃₀ Alkylacrylate crosspolymer |
| 0,25 | Hydroxyethyl cellulose |
| 8,00 | Methoxycinnamic-octylester |
| 1,00 | 4-Methylbenzylidene camphor |
| 0,50 | 5a-Tocopheryl-ascorbicacid (Ia) |
| 0,20 | Disodium EDTA |
| 5,00 | Glycerin |
| 0,15 | Fragrance |
| 0,30 | Imidazolydinyl urea |
| 0,25 | Sodium methylparaben |
| 0,15 | Sodium propylparaben |
| 5,00 | PEG-25 PABA |
| 0,10 | Sodiumhydoxide |
| ad 100 | water |

### Beispiel 3

Zusammensetzung für Feuchtigkeitscreme

| Massengehalt (Gew.-%) | |
|---|---|
| 6,00 | PEG-7-Hydrogenated castor oil |
| 5,00 | Isopropyl palmitate |
| 6,00 | Mineral oil |
| 5,00 | Jojoba oil |
| 5,00 | Mandelic oil |
| 1,00 | 5a-Tocopheryl-ascorbicacid (Ia) |
| 0,60 | Magnesiumstearate |
| 2,00 | PEG-45 / Dodecyl glycol copolymer |
| 5,00 | Glycerin |
| 0,25 | Methylparaben |
| 0,15 | Propylparaben |
| 5,00 | Imidazolydinyl urea |
| 0,15 | Fragrance |
| 0,20 | Disodium EDTA |
| ad 100 water | |

### Beispiel 4

Zusammensetzung für eine Nachtcreme ohne Konservierungsmittel

| Massengehalt (Gew.-%) | |
|---|---|
| 5,00 | PEG-7-Hydrogenated castor oil |
| 4,00 | Isopropylpalmitate |
| 4,00 | Caprylic acid / Caprinate triglyceride |
| 3,00 | 5a-Tocopheryl-ascorbicacid (Ia) |
| 1,50 | PEG-45/Dodecyl glycol copolymer |
| 0,50 | Magnesiumstearate |
| 1,50 | Dimethicone |
| 4,00 | 1,2 Propyleneglycol |
| 4,00 | Glycerin |
| 8,00 | 611 Alcohol |
| 0,15 | Fragrance |
| ad 100 | water |

### Beispiel 5

Zusammensetzung für eine Antifaltencreme

| Massengehalt (Gew.-%) | |
|---|---|
| 6,00 | PEG-7-Hydrogenated castor oil |
| 5,00 | Isopropylpalmitate |
| 10,00 | Mineral oil |
| 3,00 | Caprylic acid / Caprinate triglyceride |
| 0,60 | Magnesiumstearate |
| 1,00 | N-Aminocarbonyl-2,3-O-isopropyl-L-ketugulonicacidamide |
| 1,00 | Tocopheryl acetate |
| 2,00 | PEG-45/Dodecyl glycol copolymer |
| 0,20 | Retinol |
| 1,50 | 5a-Tocopheryl-ascorbicacid (Ia) |
| 0,30 | Glycerin |
| 0,70 | Magnesiumsulfate |
| 0,25 | Methylparaben |
| 0,15 | Propylparaben |
| 0,20 | Sodiumascorbylmonophosphate |
| 0,10 | α-Tocopherol |
| 0,10 | Ascorbyl palmitate |
| 0,15 | Fragrance |
| ad 100 | water |

### Beispiel 6

Zusammensetzung für eine Feuchtigkeits Tagescreme

| Massengehalt (Gew.-%) | |
|---|---|
| 2,00 | Ceteareth/6 |
| 2,00 | Ceteareth/25 |
| 10,00 | Mineral oil |
| 3,00 | Caprylic acid / Caprinate triglyceride |
| 3,00 | Isostearic acid |
| 3,00 | N-Aminocarbonyl-2,3-O-isopropyl-L-ketugulonicacidamide |
| 1,50 | Tocopheryl acetate |
| 2,00 | D-Panthenol USP |
| 2,50 | 5a-Tocopheryl-ascorbic acid (Ia) |
| 0,20 | Retinol |
| 0,30 | Glycerin |
| 0,15 | Dibromocyanobutane |
| 0,20 | Sodiumascorbylmonophosphate |
| 0,10 | α-Tocopherol |
| 0,10 | Ascorbylpalmitate |
| 0,15 | Fragrance |
| ad 100 | water |

## Patentansprüche

1. Chromanyl-ascorbinsäurederivate der allgemeinen Formel I 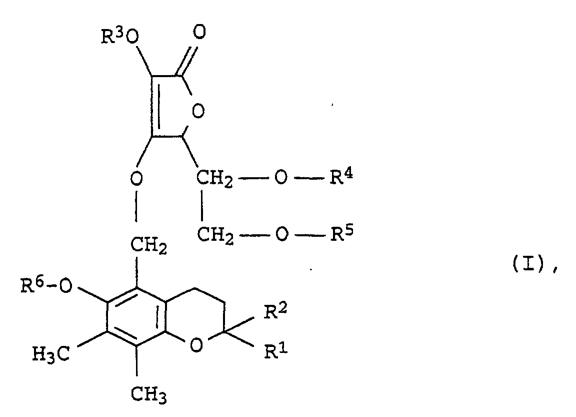 in der
R¹ für einen Kohlenwasserstoffrest mit 1 bis 4 C-Atomen steht,
R² für einen linearen oder Methyl-verzweigten gesättigten oder ungesättigten aliphatischen Kohlenwasserstoffrest mit 12 bis 24 C-Atomen steht,
R³ für Wasserstoff, eine Alkyl- oder Acylgruppe mit 1 bis 4 C-Atomen, den -PO₃H₂-Rest oder einen Glycosidylrest steht,
R⁴ und R⁵ jeweils für Wasserstoff, eine Alkyl- oder Acylgruppe mit 1 bis 4 C-Atomen stehen,
R⁶ für Wasserstoff oder eine Acylgruppe der Formel -CO-R⁷ steht
und R⁷ für einen gesättigten oder ungesättigten aliphatischen Rest mit 1 bis 20 C-Atomen oder den Phenylvinylrest steht.

2. Chromanyl-ascorbinsäurederivat der allgemeinen Formel I gemäß Anspruch 1, **dadurch gekennzeichnet, daß** R¹ für eine Methylgruppe steht,
R² für den Phytylrest 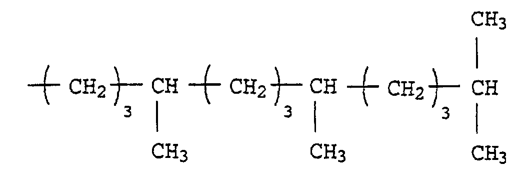 steht und R³, R⁴, R⁵ und R⁶ jeweils Wasserstoff bedeuten.

3. Verfahren zur Herstellung von Chromanyl-ascorbinsäurederivaten der allgemeinen Formel I gemäß Anspruch 1, **dadurch gekennzeichnet, daß** man ein 5-Halogenmethyl-chroman der allgemeinen Formel II 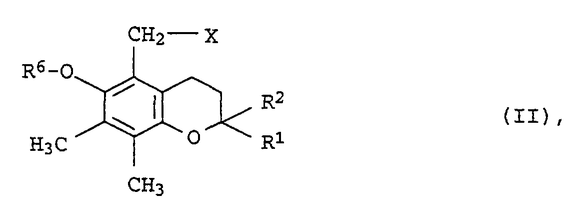 in der R^{1,} R² und R⁶ die in Anspruch 1 angegebene Bedeutung haben und X für Br oder Cl steht, mit einem Alkalisalz oder Erdalkalisalz der Ascorbinsäure oder eines Ascorbinsäurederivates der allgemeinen Formel III 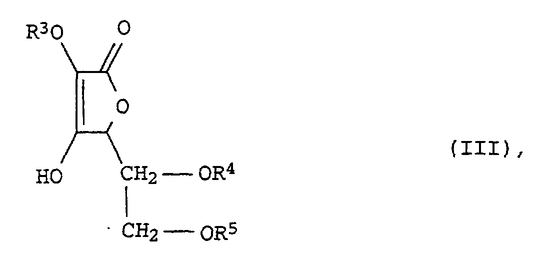 in der R³ bis R⁵ die in Anspruch 1 angegebene Bedeutung haben, umsetzt.

4. Verfahren zur Herstellung von Chromanyl-ascorbinsäurederivaten der allgemeinen Formel I nach Anspruch 3, **dadurch gekennzeichnet, daß** man die Alkalisalze oder Erdalkalisalze der Ascorbinsäure oder Ascorbinsäurederivate in situ (im Reaktionsgemisch) herstellt und anschließend umsetzt.

5. Verwendung der Chromanyl-ascorbinsäurederivate der allgemeinen Formel I gemäß Anspruch 1 zur Herstellung pharmazeutischer oder kosmetischer Zubereitungen oder von Nahrungsergänzungsmitteln.

6. Verwendung der Chromanyl-ascorbinsäurederivate nach Anspruch 5 als Bioantioxidantien.

7. Verwendung der Chromanyl-ascorbinsäurederivate der allgemeinen Formel I nach Anspruch 5 zum Stabilisieren von organischen Stoffen gegen die schädigende Einwirkung von Sauerstoff, Wärme und/oder Licht.

8. Verwendung der Chromanyl-ascorbinsäurederivate der allgemeinen Formel I nach Anspruch 5 zum Stabilisieren von Nahrungsmitteln oder pharmazeutischen oder kosmetischen Präparaten gegen die schädigende Einwirkung von Sauerstoff, Wärme und/oder Licht.

9. Verwendung der Chromanyl-ascorbinsäurederivate der allgemeinen Formel I nach Anspruch 5, **dadurch gekennzeichnet, daß** sie im Gemisch mit Ascorbinsäure oder Ascorbinsäurederivaten eingesetzt werden.

## Claims

1. A chromanylascorbic acid derivative of the general formula I 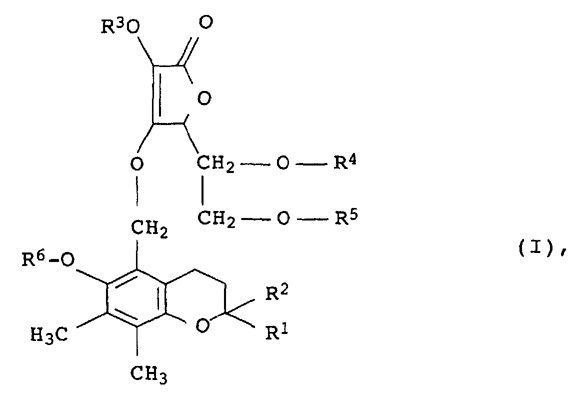 where
R¹ is a hydrocarbon radical having 1 to 4 C atoms,
R² is a linear or methyl-branched, saturated or unsaturated, aliphatic hydrocarbon radical having 12 to 24 C atoms,
R³ is hydrogen, an alkyl or acyl group having 1 to 4 C atoms, the -PO₃H₂- radical or a glycosidyl radical,
R⁴ and R⁵ are each hydrogen or an alkyl or acyl group having 1 to 4 C atoms,
R⁶ is hydrogen or an acyl group of the formula -CO-R⁷
and R⁷ is a saturated or unsaturated aliphatic radical having 1 to 20 C atoms, or the phenylvinyl radical.

2. A chromanyl ascorbic acid derivative of the general formula I as claimed in claim 1, wherein R¹ is a methyl group,
R² is the phytyl radical 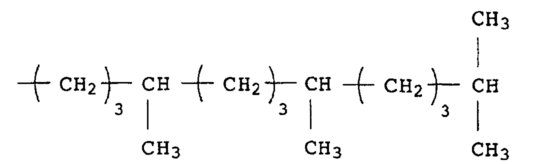 and R³, R^{4,} R⁵ and R⁶ are each hydrogen.

3. A process for the preparation of chromanylascorbic acid derivatives of the general formula I as claimed in claim 1, which comprises reacting a 5-halomethylchroman of the general formula II 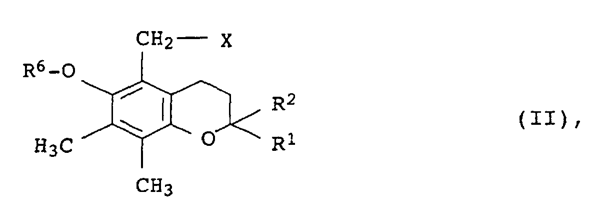 where R^{1,} R² and R⁶ have the meanings indicated in claim 1 and X is Br or Cl, with an alkali metal salt or alkaline earth metal salt of ascorbic acid or of an ascorbic acid derivative of the general formula III 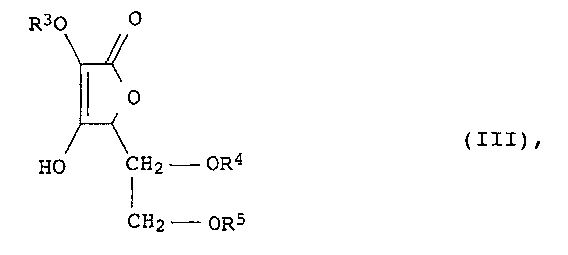 where R³ to R⁵ have the meanings indicated in claim 1.

4. A process for the preparation of chromanylascorbic acid derivatives of the general formula I as claimed in claim 3, wherein the alkali metal salts or alkaline earth metal salts of the ascorbic acid or ascorbic acid derivatives are prepared in situ (in the reaction mixture) and then reacted.

5. The use of the chromanylascorbic acid derivatives of the general formula I as claimed in claim 1 for preparing pharmaceutical or cosmetic formulations or food supplements.

6. The use of the chromanylascorbic acid derivatives as claimed in claim 5 as bioantioxidants.

7. The use of the chromanylascorbic acid derivatives of the general formula I as claimed in claim 5 for stabilizing organic substances against the harmful action of oxygen, heat and/or light.

8. The use of the chromanylascorbic acid derivatives of the general formula I as claimed in claim 5 for stabilizing foods or pharmaceutical or cosmetic preparations against the harmful action of oxygen, heat and/or light.

9. The use of the chromanylascorbic acid derivatives of the general formula I as claimed in claim 5, which comprises employing them as a mixture with ascorbic acid or ascorbic acid derivatives.

## Revendications

1. Dérivés chromanylés d'acide ascorbique de formule générale I 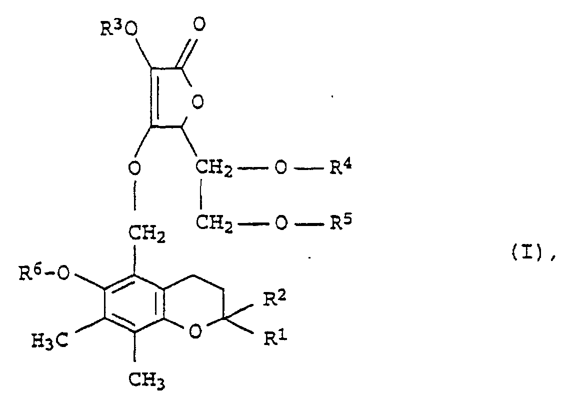 dans laquelle
R¹ désigne un reste hydrocarboné ayant 1 à 4 atomes de carbone,
R² représente un reste hydrocarboné aliphatique, saturé ou insaturé, linéaire ou à ramifications méthyle, ayant 12 à 24 atomes de carbone,
R³ désigne l'hydrogène, un groupe alkyle ou acyle ayant 1 à 4 atomes de carbone, le reste -PO₃H₂ ou un reste glycosidyle,
R⁴ et R⁵ représentent chacun l'hydrogène, ou un groupe alkyle ou acyle ayant 1 à 4 atomes de carbone,
R⁶ désigne l'hydrogène ou un groupe acyle de formule -CO-R⁷
et R⁷ représente un reste aliphatique saturé ou insaturé ayant 1 à 20 atomes de carbone ou le reste phénylvinyle.

2. Dérivé chromanylé d'acide ascorbique de formule générale I selon la revendication 1, **caractérisé par le fait que** R¹ représente un groupe méthyle,
R² désigne le reste phytyle 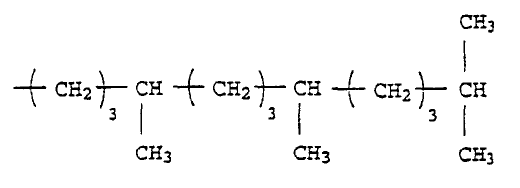 et R³, R⁴, R⁵ et R⁶ représentent chacun l'hydrogène.

3. Procédé pour la préparation de dérivés chromanylés d'acide ascorbique de formule générale I selon la revendication 1, **caractérisé par le fait qu'**on fait réagir un 5-halogénométhyl-chromane de formule générale II 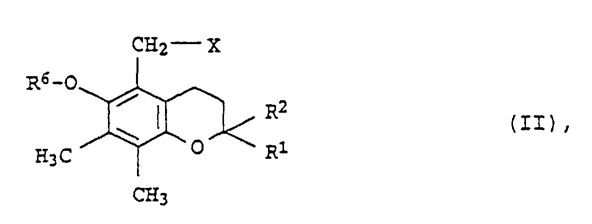 dans laquelle R¹, R² et R⁶ ont la signification indiquée dans la revendication 1, et X désigne Br ou Cl, avec un sel alcalin ou un sel alcalino-terreux d'acide ascorbique ou un dérivé d'acide ascorbique de formule générale III 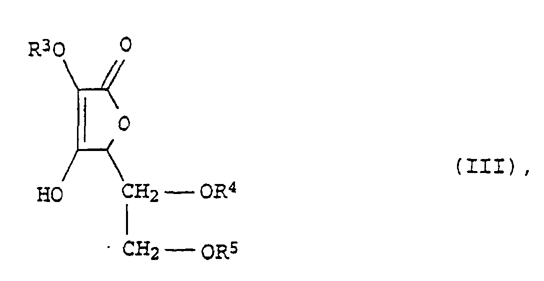 dans laquelle R³ à R⁵ ont la signification indiquée dans la revendication 1.

4. Procédé pour la préparation de dérivés chromanylés d'acide ascorbique, de formule générale I selon la revendication 3, **caractérisé par le fait qu'**on prépare les sels alcalins ou les sels alcalino-terreux d'acide ascorbique ou de dérivés d'acide ascorbique in situ (dans le mélange réactionnel) et on les transforme ensuite.

5. Utilisation des dérivés chromanylés d'acide ascorbique de formule générale I selon la revendication 1 pour l'obtention de préparations pharmaceutiques ou cosmétiques ou de compléments alimentaires.

6. Utilisation des dérivés chromanylés d'acide ascorbique selon la revendication 5 comme bioantioxydants.

7. Utilisation des dérivés chromanylés d'acide ascorbique de formule générale I selon la revendication 5 pour la stabilisation de substances organiques contre l'influence néfaste de l'oxygène, de la chaleur et/ou de la lumière.

8. Utilisation des dérivés chromanylés d'acide ascorbique de formule générale I selon la revendication 5 pour la stabilisation de produits alimentaires ou de préparations pharmaceutiques ou cosmétiques contre l'influence néfaste de l'oxygène, de la chaleur et/ou de la lumière.

9. Utilisation des dérivés chromanylés d'acide ascorbique de formule générale I selon la revendication 5, **caractérisée par le fait qu'**ils sont mis en oeuvre en mélange avec de l'acide ascorbique ou des dérivés d'acide ascorbique.
